# EUROPÄISCHE PATENTSCHRIFT

(11) **EP 1 043 332 B1**
(45) Veröffentlichungstag und Bekanntmachung des Hinweises auf die Patenterteilung: **02.11.2006**
(21) Anmeldenummer: 00110958.6
(22) Anmeldetag: 08.07.1996
(51) Int. Cl.: C07K 5/06, C07C 229/48

(54) **Cyclopentan-beta-Aminosäuren enthaltende Dipeptide**
Dipeptides comprising cyclopentan-beta-amino acids
Dipeptides contenant d'acid aminés beta cyclopentane

(30) Priorität: 19.07.1995 DE 19526274
(43) Veröffentlichungstag der Anmeldung: 11.10.2000
(62) Teilanmeldung aus: 96110967.5
(73) Patentinhaber: Bayer HealthCare AG, 51368 Leverkusen (DE)
(72) Erfinder: Matzke, Michael, Dr., 42113 Wuppertal (DE); Militzer, Hans-Christian, Dr., 51467 Bergisch Gladbach (DE); Mittendorf, Joachim, Dr., 42113 Wuppertal (DE); Kunisch, Franz, Dr., 51519 Odenthal (DE); Schmidt, Axel, Dr., 42327 Wuppertal (DE); Schönfeld, Wolfgang, Dr., 10000 Zagreb (HU); Ziegelbauer, Karl, Dr., 42781 Haan (DE)

(56) Entgegenhaltungen:
- EP-A- 0 571 870
- WO-A-95/07022
- DE-A- 19 548 825
- DE-A- 19 604 225
- DATABASE WPI Section Ch, Week 9033 Derwent Publications Ltd., London, GB; Class B05, AN 90-250724 XP002042215 & JP 02 174753 A (FUJISAWA PHARM CO LTD), 6. Juli 1990 (1990-07-06)
- OHKI HIDENORI ET AL.: "Synthesis and antifungal activity of FR 109615 Analogs" J. ANTIBIOT., Bd. 44, Nr. 5, 1991, Seiten 546-549, XP002042039

## Beschreibung

Aus den Publikationen EP-A-571 870, DOS 43 02 155, JP 021 747 53 A2 und J. Antibiot. (1991), 44 (5), 546-9 sind Cyclopentan- und -penten-β-aminosäuren bekannt. Solche β-Aminosäureverbindungen wirken antimikrobiell, insbesondere antimykotisch. Sie sind jedoch nicht frei von Nebenwirkungen.

Überraschenderweise wurde nun gefunden, dass Dipeptide aus α-Aminosäuren und/oder deren Derivaten und Cyclopentan-β-aminosäuren und/oder deren Derivaten, diese unerwünschten Nebenwirkungen nicht oder nur in geringerem Ausmaß aufweisen und damit eine verbesserte Verträglichkeit bei Warmblütern erreicht wird. Die Bezeichnung "Derivate" umfasst solche Verbindungen, die sich von den entsprechenden Aminosäuren ableiten und eine vergleichbare Wirkung aufweisen, insbesondere die entsprechenden Salze.

Gegenstand der vorliegenden Erfindung sind Dipeptide bestehend aus einer α-Aminosäure oder einem Derivat davon und einer Cyclopentan-β-aminosäure oder einem Derivat davon.

Die vorliegende Erfindung betrifft Dipeptide bestehend aus einer α-Aminosäure der allgemeinen Formel (Ia), 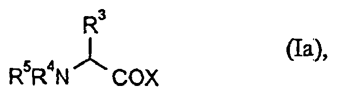 in welcher
R³ für Methyl oder für eine Gruppe der Formel -CH(CH₃)CH₂CH₃ steht,
R⁴ und R⁵ für Wasserstoff stehen oder
R³ und R⁴ gemeinsam einen Rest der Formel -(CH₂)₃- bilden,
R⁵ für Wasserstoff steht und
X für den Anteil der kovalenten Bindung der α-Aminosäure und der Cyclopentan-β-aminosäure steht,
und einer Cyclopentan-β-aminosäure der allgemeinen Formel (Ib), 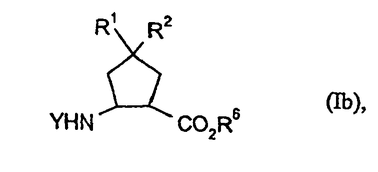 in welcher
R¹ und R² für Wasserstoff stehen oder
R¹ und R² gemeinsam einen Rest der Formel =CH₂ bilden,

### R⁶ für Wasserstoff steht und

Y für den Anteil der kovalenten Bindung der α-Aminosäure und der Cyclopentan-β-aminosäure steht.

Ganz besonders bevorzugt sind folgende Dipeptide:
1,2-cis-2-(S)-Isoleucyl-amino-4-methylencyclopentan-1-carbonsäure und
1,2-cis-2-(S)-Alanyl-amino-4-methylencyclopentan-1-carbonsäure.

Die erfindungsgemäßen Dipeptide können aus im wesentlichen reinen Stereoisomeren oder Stereoisomerengemische bestehen.

Die vorstehend beschriebenen α-Aminosäuren, Cyclopentan-β-aminosäuren und Dipeptide können auch in Form ihrer Salze vorliegen. Im allgemeinen seien hier Salze mit organischen oder anorganischen Basen oder Säuren sowie innere Salze genannt.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin, Ethylendiamin oder Phenethylamin.

Die erfindungsgemäßen Dipeptide können in stereoisomeren Formen existieren, beispielsweise entweder wie Bild und Spiegelbild (Enantiomere), oder die sich nicht wie Bild und Spiegelbild (Diastereomere) verhalten, beziehungsweise als Diastereomerengemisch oder als reine cis- oder trans-Isomere vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen. Die Trennung in die stereoisomer einheitlichen Verbindungen erfolgt beispielsweise über eine chromatographische Racematspaltung von diastereomeren Estern und Amiden oder an optisch aktiven Phasen. Außerdem ist eine Kristallisation von diastereomeren Salzen möglich.

Im Rahmen der Erfindung liegen die durch den Rest (R⁵R⁴-N-CHR³-CO-) definierten Aminosäurereste in der L-Form vor.

Ebenfalls Gegenstand der vorliegenden Erfindung ist ein Verfahren zur Herstellung der erfindungsgemäßen Dipeptide.

Diese können hergestellt werden, indem man Verbindungen der allgemeinen Formel (II) 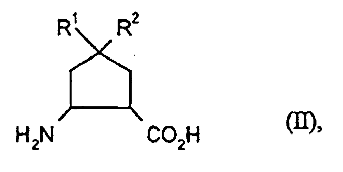 in welcher
R¹ und R² die oben angegebenen Bedeutungen haben,
durch Umsetzung mit geschützten Aminosäuren der allgemeinen Formel (III) 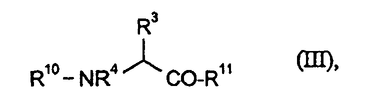 in welcher
R³ und R⁴ die oben angegebenen Bedeutungen haben,
R¹⁰ für eine Aminoschutzgruppe steht
und
R¹¹ für eine in der Peptidchemie übliche aktivierende Schutzgruppe, vorzugsweise für den Hydroxysuccinimidesterrest steht oder
R¹⁰ und R¹¹ zusammen für die Gruppierung 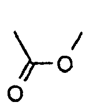 stehen,
zunächst in die Verbindungen der allgemeinen Formel (IV) 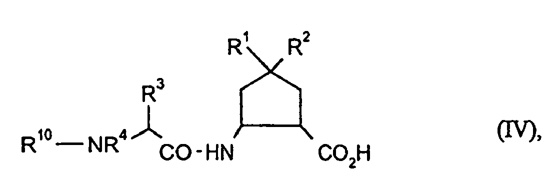 in welcher
R¹, R², R³, R⁴ und R¹⁰ die oben angegebenen Bedeutungen haben,
in Lösemitteln und in Anwesenheit einer Base überfuhrt,
und abschließend die Aminoschutzgruppe (R¹⁰) abspaltet,
gegebenenfalls die Stereoisomeren trennt, und im Fall der Ester (R⁶ ≠ H in Formel (Ib)) die Säuren nach üblichen Methoden mit den entsprechenden Alkoholen umsetzt.

Gegebenenfalls werden die Dipeptide nach üblichen Methoden in die Salze überführt.

Das erfindungsgemäße Verfahren kann durch folgendes Formelschema beispielhaft erläutert werden:

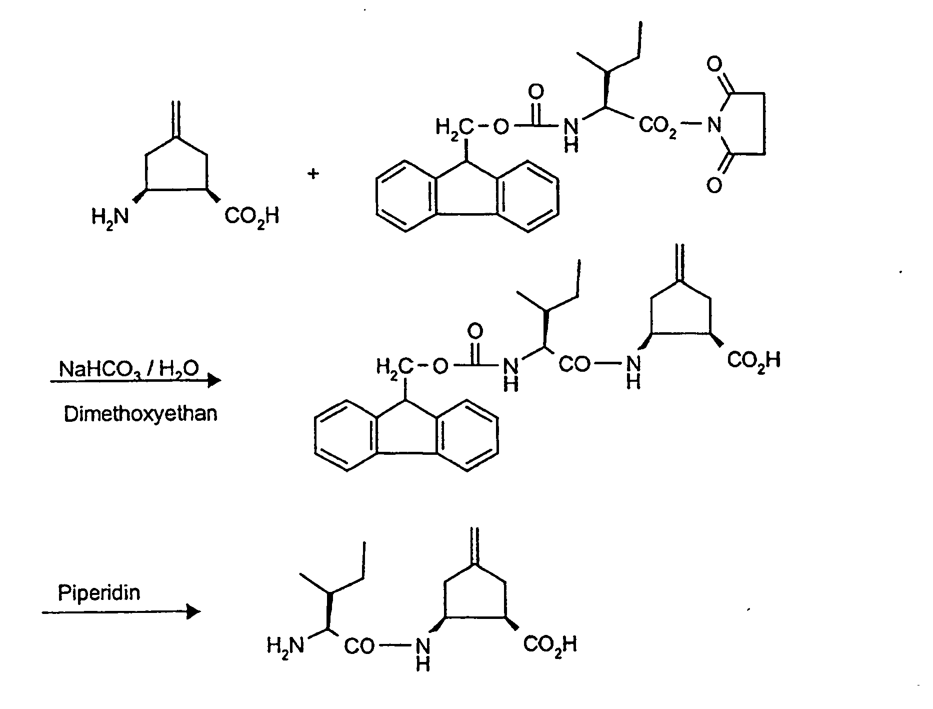

Aminoschutzgruppen (R¹⁰) im Rahmen der Erfindung sind die üblichen in der PeptidChemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, tert.Butoxycarbonyl, Allyloxycarbonyl, Vinyloxycarbonyl, 3,4,5-Trimethoxybenzyloxycarbonyl, Phthaloyl, 2,2,2-Trichlorethoxycarbonyl, 2,2,2-Trichlor-tertbutoxycarbonyl, Menthyloxycarbonyl, 4-Nitrophenoxycarbonyl, Fluorenyl-9-methoxycarbonyl (Fmoc), Formyl, Acetyl, Propionyl, Pivaloyl, 2-Chloracetyl, 2-Bromacetyl, 2,2,2-Trifluoracetyl, 2,2,2-Trichloracetyl, Benzoyl, Benzyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl oder 2-Nitrophenylsulfenyl. Besonders bevorzugt ist die Fmoc-Gruppe.

Als aktivierende Carboxylreste (R¹¹) eignen sich im allgemeinen Addukte mit Carbodiimiden z.B. N,N-Diethyl-, N,N-Diisopropyl-, N,N-Dicyclohexylcarbodiimid, N-(3-Dimethylaminoisopropyl)-N'-ethyl-carbodiimid-Hydrochlorid, N-Cyclohexyl-N-(2-morphlinoethyl)-carbodiimid-metho-p-toluolsulfonat, oder Carbonylverbindungen wie Carbonyldiimidazol, oder 1,2-Oxazoliumverbindungen wie 2-Ethyl-5-phenyl-1,2-oxazolium-3-sulfat oder 2-tert.Butyl-5-methyl-isoxazolium-perchlorat, oder Acylaminoverbindungen wie 2-Ethoxy-1-ethoxycarbcnyl-1,2-dihydrochinolin, oder Propanphosphonsäureanhydrid, oder Isobutylehloroformat, oder Benzotriazolyloxy-tris-(dimethylamino)phosphonium-hexafluorophosphat, 1-Hydroxybenzotriazol oder Hydroxysuccinimidester. Weiterhin kann die α-Aminosäurekomponente auch in Form eines Leuch'schen Anhydrids eingesetzt werden (R¹⁰ und R¹¹ stehen in Formel (III) zusammen für die Gruppierung 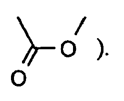 Bevorzugt ist der Hydroxysuccinimidester.

Als Lösemittel eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, Dimethoxyethan oder Kohlenwasserstoffe wie Benzol, Toluol, Xylol, Hexan, Cyclohexan, oder Erdölfraktionen oder Dimethylformamid. Ebenso ist es möglich, Gemische der genannten Lösemittel zu verwenden. Bevorzugt sind Tetrahydrofuran, Diethylether und Dimethoxyethan. Außerdem ist es möglich, Wasser oder Gemische der oben aufgeführten Lösemittel mit Wasser einzusetzen.

Außerdem können beispielsweise Alkalicarbonate, z.B. Natrium- oder Kaliumcarbonat oder -hydrogencarbonat, oder organische Basen wie Trialkylamine, z.B. Triethylamin, Ethyldiisopropylamin, N-Ethylmorpholin, N-Methylpiperidin oder N-Methylmorpholin eingesetzt werden. Bevorzugt ist N-Methylmorpholin.

Die Hilfsstoffe und Basen werden in einer Menge von 1,0 mol bis 3,0 mol, bevorzugt 1,0 mol bis 1,2 mol, bezogen auf 1 mol der Verbindungen der allgemeinen Formel (III), eingesetzt.

Die Reaktionen werden in einem Temperaturbereich von 0°C bis 100°C, vorzugsweise bei 0°C bis 30°C und bei Normaldruck durchgeführt.

Die Reaktionen können sowohl bei Normaldruck als auch bei erhöhtem oder erniedrigtem Druck (beispielsweise 0,5 bis 5 bar), vorzugsweise bei Normaldruck durchgeführt werden.

Die Abspaltung der Aminoschutzgruppe erfolgt im allgemeinen in an sich bekannter Weise unter sauren oder basischen Bedingungen, oder reduktiv durch katalytische Hydrierung beispielsweise mit Pd/C in organischen Lösemitteln wie Ethern, z.B. Tetrahydrofuran oder Dioxan, oder Alkoholen z.B. Methanol, Ethanol oder Isopropanol.

Die Hydrierung erfolgt im allgemeinen in einem Temperaturbereich von 0°C bis 80°C, vorzugsweise von 0°C bis 40°C.

Im allgemeinen wird die Hydrierung bei erhöhtem Druck von 2 bar bis 8 bar, vorzugsweise von 3 bis 5 bar, durchgeführt.

Für die Abspaltung der Aminoschutzgruppe (R¹⁰ = Fmoc) eignen sich Basen wie beispielsweise Piperidin, Morpholin, Dicyclohexylamin, p-Dimethylaminopyridin, Diisopropylethylamin oder Piperazin. Bevorzugt ist Piperidin.

Die Hilfsstoffe und Basen werden in einer Menge von 1,0 mol bis 3,0 mol, bevorzugt 1,0 mol bis 1,2 mol, bezogen auf 1 mol der Verbindungen der allgemeinen Formel (IV), eingesetzt.

Die Reaktionen werden in einem Temperaturbereich von 0°C bis 100°C, vorzugsweise bei 0°C bis 30°C und bei Normaldruck durchgeführt.

Die Reaktionen können sowohl bei Normaldruck als auch bei erhöhtem oder erniedrigtem Druck (beispielsweise 0,5 bis 5 bar), vorzugsweise bei Normaldruck durchgeführt werden.

Die Verbindungen der allgemeinen Formel (II) sind bekannt.

Die Verbindungen der allgemeinen Formel (III) sind teilweise bekannt oder nach üblichen Methoden herstellbar.

Die vorstehenden Herstellungsverfahren sind lediglich zur Verdeutlichung angegeben. Die Herstellung der erfindungsgemäßen Verbindungen der allgemeinen Formeln (Ia) und (Ib), in denen X und Y zusammen für eine kovalente Bindung stehen, ist nicht auf diese Verfahren beschränkt, jede Modifikation dieser Verfahren ist in gleicher Weise für die Herstellung anwendbar.

Ausgangspunkt für die vorliegende Erfindung war die Aufklärung des folgenden Mechanismus:
Die hier beschriebenen Cyclopentan-β-aminosäuren der allgemeinen Formel (II) werden von verschiedenen Hefespecies durch Aminosäuretransporter akkumuliert. Der Transport der β-Aminosäuren kann durch aliphatische Aminosäuren, insbesondere L-Isoleucin, L-Leucin, L-Alanin, L-Methionin und L-Valin, gehemmt werden. β-Aminosäuren hemmen die Proteinbiosynthese. Diese Hemmung kann durch eine der aliphatischen Aminosäuren, insbesondere durch L-Isoleucin oder L-Alanin, antagonisiert werden. Die gleichzeitige Gabe von β-Aminosäure und der antagonisierenden natürlichen Aminosäure als kovalent verknüpft als Dipeptid führt zur Verringerung der bei Warmblütern auftretenden Nebenwirkungen bei gleichzeitiger Erhaltung der antimykotischen Wirkung in vivo.
Die erfindungsgemäßen Verbindungen zeigen daher ein nicht vorhersehbares, wertvolles pharmakologisches Wirkspektrum.
Die erfindungsgemäßen Verbindungen und ihre Säureadditions-Salze weisen in vivo antimikrobielle, insbesondere starke antimykotische Wirkungen auf. Gleichzeitig bedingt ihre geringere Toxizität eine bessere Verträglichkeit. Sie besitzen ein breites antimykotisches Wirkspektrum gegen Dermatophyten, wie Trichophyton mentagrophytes und Microsporum canis, gegen Sproßpilze wie Candida albicans, Candida glabrata, Epidermophyton floccosum und gegen Schimmelpilze wie Aspergillus niger und Aspergillus fumigatus. Die Aufzählung dieser Mikroorganismen stellt keinesfalls eine Beschränkung der bekämpfbaren Keime dar, sondern hat nur erläuternden Charakter. Sie sind deshalb geeignet zur Behandlung von Dermatomykosen und Systemmykosen.

### Prüfung der In-vivo-Wirksamkeit

Als Testmodell für antimykotische In-vivo-Wirkungen wurde die systemische Mäuse-Candidose benutzt:
Männliche CFW₁-Mäuse von 20 g Gewicht wurden durch Injektion von 3 x 10⁵ KBE von C. albicans pro Tier in die Schwanzvene infiziert.
Unbehandelte Kontrolltiere sterben vollständig innerhalb einer Woche post infectionem (p.i.) an einer generalisierten Candidose mit Granulombildung in der Niere. Zur Wirksamkeitsprüfung wurden die Präparate, gelöst in einer 0,2%igen wässrigen Glucose-Agar-Lösung, den infizierten Tieren 2 mal täglich oral per Schlundsonde appliziert.

Die Tagesdosen betrugen 2 x 25 mg/kg und 2 x 50 mg/kg Körpergewicht (KG), die Therapiedauer betrug 5 Tage.

Die Überlebensraten der behandelten Tiere wurden täglich bis zum 10. Tag p.i. notiert. Zu diesem Zeitpunkt überlebten bei den unbehandelten Kontrolltieren keine Tiere.

Für die Präparate wurden pro Dosis- und Kontrollgruppe je 10 Tiere eingesetzt.

Die Ergebnisse sind in Tabelle A aufgeführt.

**Tabelle A**

| **Bsp.-Nr.** | **Dosis [mg/kg, 2 x tägl.]** | **Applikation** | **Zahl der überlebenden Tiere** |
|---|---|---|---|
| Kontrolle | | | 0/10 |
| 2 | 25 | p.o. | 6/10 |
| 2 | 50 | p.o. | 10/10 |

Alternativ kann die In-vivo-Wirksamkeit auch an Wistar-Ratten getestet werden. Diese würden geringere Tagesdosen, bezogen auf mg/kg KG, benötigen, um einen vergleichbaren Therapieeffekt zu erzielen. Der Test wird in diesem Fall wie folgt durchgeführt:
Acht Wochen alte, spezifisch pathogenfreie, männliche Wistar-Ratten von 200 g werden mit 5 x 10⁶ KBE Candida albicans in 0,5 ml PBS über die laterale Schwanzvene infiziert. Dieses führt zu einer 100 %igen Mortalität innerhalb von acht Tagen.

Die Tiere zeigen schon einen Tag nach Infektion Blutungen im medialen Augenwinkel; neben der Niere werden andere Organsysteme wie Hirn, Herz, Leber, Milz, Retina und Lunge befallen. Substanzapplikation erfolgt zweimal täglich über fünf Tage peroral in je 1 ml Glucose (5%)-Agar (0,2%)-Lösung beginnend am Tag der Infektion.

Die bessere Verträglichkeit der erfindungsgemäßen Dipeptide wurde auf die folgende Weise getestet:
Wistar-Ratten wurden täglich mit den entsprechenden Substanzen gefüttert und der Gewichtsverlauf protokolliert. Es wurde entweder die β-Aminosäure alleine oder eine äquimolare Menge des entsprechenden Dipeptids mit einer α-Aminosäure verabreicht. Nach 5 Tagen Behandlungsdauer war das Körpergewicht der Ratten bei Gabe der erfindungsgemäßen Dipeptide gleich geblieben oder leicht gestiegen, während es bei der Behandlung mit der β-Aminosäure um etwa 5 bis 10 % abgenommen hatte.
Zur vorliegenden Erfindung gehören auch Arzneimittel, enthaltend die erfindungsgemäßen Dipeptide sowie nicht-toxische, inerte pharmazeutische Träger- und Hilfsstoffe zur Bekämpfung von Krankheiten, insbesondere von Mykosen.
Der oder die Wirkstoffe können gegebenenfalls in einem oder mehreren der oben angegebenen Trägerstoffe auch in mikroverkapselter Form vorliegen.
Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.
Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.
Die oben aufgeführten pharmazeutischen Zubereitungen können außer den erfindungsgemäßen Verbindungen auch weitere pharmazeutische Wirkstoffe enthalten.
Die Wirkstoffe oder die Arzneimittel können oral und parenteral, appliziert werden.
Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.
Bei den erfindungsgemäßen Arzneimitteln handelt es sich üblicherweise um Kombinationspräparate zur gleichzeitigen, getrennten oder zeitlich abgestuften Anwendung bei der Bekämpfung von Krankheiten.
Bei Kombinationspräparaten zur gleichzeitigen Anwendung handelt es sich um Erzeugnisse, in denen die Einzelkomponenten der erfindungsgemäßen Gemische als physikalische Mischung vorliegen. Hierzu zählen insbesondere Tabletten, Dragees, Kapseln, Pillen, Suppositorien und Ampullen. Ebenso denkbar ist der Einsatz derartiger Mischungen als Lösung, Suspension oder Emulsion.
Bei Kombinationspräparaten zur getrennten Anwendung handelt es sich um Erzeugnisse, in denen die Einzelkomponenten der erfindungsgemäßen Gemische räumlich
voneinander getrennt vorliegen. Hierzu eignen sich insbesondere Tabletten, Dragees, Kapseln, Pillen und Suppositorien, die dieser Anforderung gerecht werden.

Ebenso denkbar sind Kombinationspräparate zur zeitlich abgestuften Anwendung.

### Ausgangsverbindungen

### Beispiel I

### (-)-1,2-cis-2-((N-(9-Fluorenylmethyloxycarbonyl)-(S)-isoleucyl)-amino-4-methylencyclopentan-1-carbonsäure

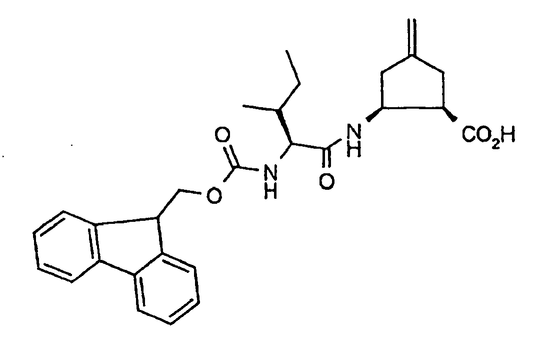

Zu einer Lösung von (-)-1,2-cis-2-Amino-4-methylen-cyclopentan-1-carbonsäure (35,1 g, 0,198 mol) und Natriumhydrogencarbonat (33,36 g, 0,397 mol) in 480 ml Wasser wird bei Raumtemperatur eine Lösung von N-(9-Fluorenylmethyloxycarbonyl)-(S)-isoleucin-Hydroxysuccinimidester (89,2 g, 0,198 mol) in 600 ml Dimethoxyethan zugetropft. Es wird über Nacht bei Raumtemperatur gerührt. Der Reaktionsansatz wird anschließend mit verdünnter Salzsäure auf pH 2 angesäuert und mehrfach mit Diethylether extrahiert. Die vereinigten organischen Phasen werden über Natriumsulfat getrocknet und im Vakuum eingeeengt. Das Produkt wird aus Diethylether / Petrolether kristallisiert.
Ausbeute: 70 g (74% d.Th.)
Schmp.: 207°C
[α]²⁰_{D}= -24,1 (c=1,15 in Chloroform)
¹H-NMR (250 MHz, CDCl₃): δ = 0,88 (cm, 6H); 0,98 - 1,15, 1,40 - 1,51, 1,52 - 1,80
(3m, 3H); 2,40 - 2,84 (m, 4H); 3,12 (cm, 1H); 4,10 - 4,48 (m, 4H); 4,61 (cm, 1H); 4,90 (cm, 2H); 5,84 (d, 1H); 7,20 - 7,80 (3m, 9H).
C₂₈H₃₂N₂O₅ (476,6)

### Beispiel II

### 1,2-cis-2-(N-(9-Fluorenylmethyloxycarbonyl)-(S)-alanyl)amino-4-methylen-cyclopentan-1-carbonsäure

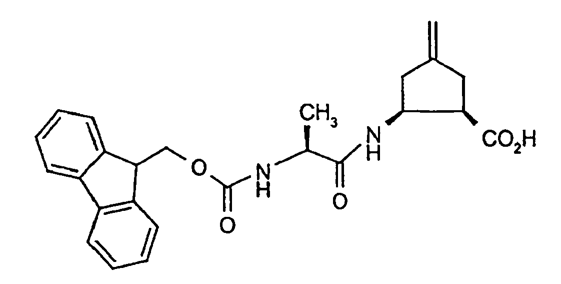

Die Titelverbindung wird analog zu der Vorschrift für Beispiel I aus (-)-1,2-cis-2-Amino-4-methylen-cyclopentan-1-carbonsäure (2,27 g, 16,1 mmol), N-(9-Fluorenyl-methyloxycarbonyl)-(S)-alanin-Hydroxysuccinimidester (7,0 g, 17,2 mmol) und Natriumhydrogencarbonat (1,49 g, 17,7 mmol) dargestellt. Das Rohprodukt wird säulenchromatographisch gereinigt (Toluol/ Ethanol, 9:1).
Ausbeute: 5,7 g (81% d.Th.)
¹H-NMR (500 MHz, CD₃OD): δ = 1.30 (d, 3H), 2.43 - 2.79 (m, 4H), 3.10 (cm, 1H), 4.12, 4.21, 4.34, 4.50 (4 cm, 5H), 4.91 (br. s., 2H), 7.39, 7.3, 7.66, 7.79 (4 cm, 8H).
C₂₅H₂₆N₂O₅ (434,5)

### Herstellungsbeispiele

### Beispiel 1

### (+)-1,2-cis-2-(S)-Isoleucyl-amino-4-methylen-cyclopentan-1-carbonsäure

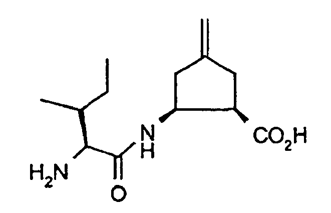

Eine Lösung der Verbindung aus Beispiel I (24,0 g , 0,050 mol) in Piperidin (200 ml) wird 1 h bei Raumtemperatur gerührt. Nach Beendigung der Reaktion wird das Piperidin im Vakuum abdestilliert. Der Rückstand wird in Wasser aufgenommen. Nach mehrfacher Extraktion mit Diethylether wird die wässrige Phase unter Zugabe von Toluol im Vakuum eingeengt. Das Produkt wird aus Isopropanol / Diethylether kristallisiert.
Ausbeute: 8,5 g (67% d.Th.)
Schmp.: 198°C
[α]²⁰_{D} = +23,9 (c=1,08 in Wasser)
¹H-NMR (250 MHz, D₂O): δ = 0,70 - 0,88 (m, 6H); 0,91 - 1,18, 1,19 - 1,43,1,53 -1,72 (3m, 3H); 2,23 - 2,67 (m, 4H); 2,88 (cm, 1H); 3,28 (d, 1H); 4,30 (cm, 1H); 4,85 (cm, 2H).
C₁₃H₂₂N₂O₃ (254,3)

### Beispiel 2

(+)-1,2-cis-2-(S)-Alanyl-amino-4-methylen-cyclopentan-1-carbonsäure 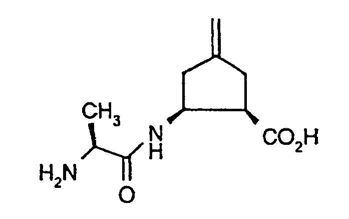 Die Titelverbindung wird analog zu der Vorschrift von Beispiel 1 aus Beispiel II (5,7 g, 13,1 mmol) dargestellt. Das Produkt wird säulenchromatographisch über Kieselgel gereinigt (Dichlormethan/Methanol, 1:1) und aus Methanol/Isopropanol/Aceton kristallisiert.
Ausbeute: 0.7 g (25% d.Th.)
Schmp. 218°C
[α]_{D}²⁰ = +5,4 (c = 0,64 in Methanol)
¹H-NMR (500 MHz, D₂O): δ = 1.49 (d, 3H), 2.45 (cm, 1H), 2.55 - 2.75 (m, 3H), 3.04 (cm, 1H), 4.01 (q, 1H), 4,49 (cm, 1H), 5.00 (br, d, 2H)
C₁₀H₁₆N₂O₃ (212,3)

## Patentansprüche

1. Dipeptid bestehend aus einer α-Aminosäure der allgemeinen Formel (Ia) 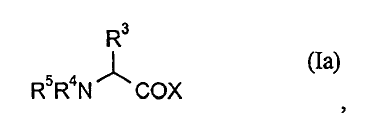 und einer Cyclopentan-β-Aminosäure der allgemeinen Formel Ib) 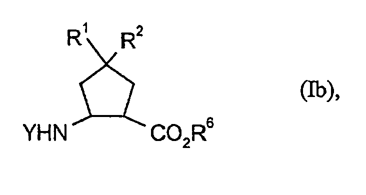 worin
R³ für Methyl oder für eine Gruppe der Formel -CH(CH₃)CH₂CH₃ steht,
R⁴ und R⁵ für Wasserstoff stehen oder
R³ und R⁴ gemeinsam einen Rest der Formel -(CH₂)₃- bilden,
R⁵ für Wasserstoff steht und
X für den Anteil der kovalenten Bindung der α-Aminosäure und der Cyclopentan-β-aminosäure steht,
R¹ und R² für Wasserstoff stehen oder
R¹ und R² gemeinsam einen Rest der Formel =CH₂ bilden,
R⁶ für Wasserstoff steht und
Y für den Anteil der kovalenten Bindung der α-Aminosäure und der Cyclopentan-β-aminosäure steht,
und deren Salze.

2. Dipeptid gemäß Anspruch 1 ausgewählt aus der Gruppe bestehend aus:
(a) 1,2-cis-2-(S)-Isoleucyl-amino-4-methylencyclopentan-1-carbonsäure und
(b) 1,2-cis-2-(S)-Alanyl-amino-4-methylencyclopentan-1-carbonsäure und deren Salze.

3. Verfahren zur Herstellung von Dipeptiden gemäß einem der Ansprüche 1 oder 2, **dadurch gekennzeichnet, dass** man Verbindungen der allgemeinen Formel (II) 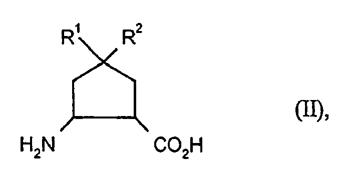 in welcher
R¹ und R² die oben angegebenen Bedeutungen haben,
durch Umsetzung mit geschützten Aminosäuren der allgemeinen Formel (III) 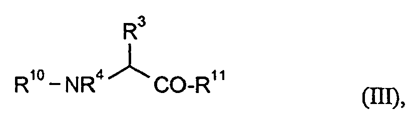 in welcher
R³ und R⁴ die oben angegebene Bedeutung haben,
R¹⁰ für eine Aminoschutzgruppe steht
und
R¹¹ für eine in der Peptidchemie übliche aktivierende Schutzgruppe, vorzugsweise für den Hydroxysuccinimidesterrest steht oder
R¹⁰ und R¹¹ zusammen für die Gruppierung 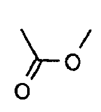 stehen,
zunächst in die Verbindungen der allgemeinen Formel (IV) 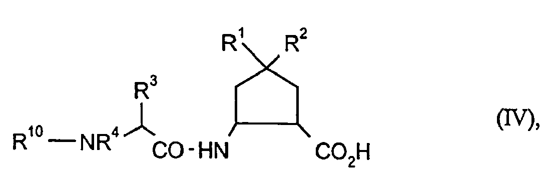 in welcher
R¹, R², R³, R⁴ und R¹⁰ die oben angegebenen Bedeutungen haben,
in Lösemitteln und in Anwesenheit einer Base überführt,
und anschließend die Aminoschutzgrüppe (R¹⁰) abspaltet, gegebenenfalls die Stereoisomeren trennt, im Fall der Ester (R⁶ ≠ H in Formel (Ib)) die Säuren nach üblichen Methoden mit den entsprechenden Alkoholen umsetzt und dass man gegebenenfalls die Dipeptide in ihre Salze überführt.

4. Dipeptide nach einem der Ansprüche 1 oder 2 zur Behandlung von Krankheiten, insbesondere von Mykosen.

5. Arzneimittel, enthaltend Dipeptide nach einem der Ansprüche 1 oder 2 zur Bekämpfung von Krankheiten, insbesondere von Mykosen.

6. Verwendung von Dipetiden nach einem der Ansprüche 1 oder 2 zur Herstellung von Arzneimitteln, insbesondere zur Bekämpfung von Mykosen.

## Claims

1. Dipeptide consisting of an α-amino acid of the general formula (Ia) 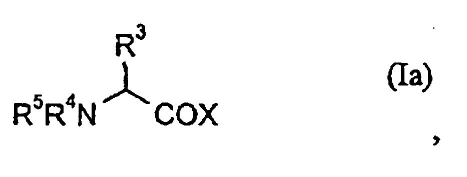 and a cyclopentane-β-amino acid of the general formula (Ib) 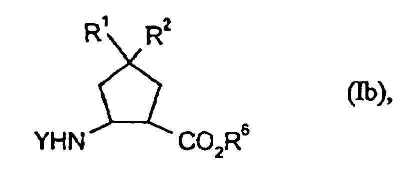 in which
R³ represents methyl, or a group of the formula -CH(CH₃)CH₂CH₃,
R⁴ and R⁵ represent hydrogen or
R³ and R⁴ together form a radical of the formula -(CH₂)₃-,
R⁵ represents hydrogen and
X represents the proportion of the covalent bond of the α-amino acid and of the cyclopentane-β-amino acid,
R¹ and R² are hydrogen or
R¹ and R² together form a radical of the formula =CH₂,
R⁶ represents hydrogen and
Y represents the proportion of the covalent bond of the α-amino acid and of the cyclopentane-β-amino acid
and salts thereof.

2. Dipeptide according to Claim 1 selected from the group consisting of:
(a) 1,2-cis-2-(S)-isoleucylamino-4-methylenecyclopentane-1-carboxylic acid and
(b) 1,2-cis-2-(S)-alanylamino-4-methylenecyclopentane-1-carboxylic acid and salts thereof.

3. Process for the preparation of dipeptides according to Claim 1 or 2, **characterized in that** compounds of the general formula (II) 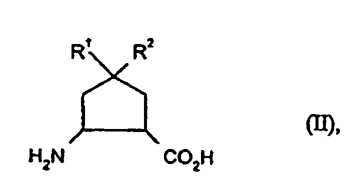 in which
R¹ and R² have the abovementioned meanings,
are first converted, by reaction with protected amino acids of the general formula (III) 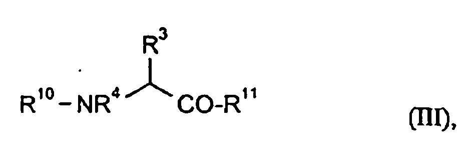 in which
R³ and R⁴ have the abovementioned meanings,
R¹⁰ represents an amino-protective group
and
R¹¹ represents an activating protective group which is customary in peptide chemistry, preferably the hydroxysuccinimide ester radical, or
R¹⁰ and R¹¹ together represent the grouping 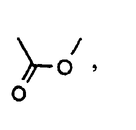 into the compounds of the general formula (IV) 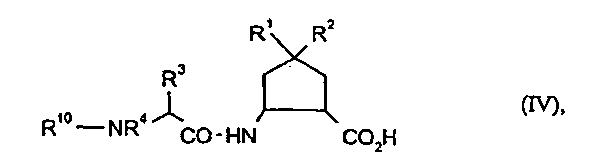 R¹, R², R³, R⁴ and R¹⁰ have the abovementioned meanings,
in solvents and in the presence of a base,
and, finally, the amino-protective group (R¹⁰) is split off,
if appropriate the stereoisomers are separated, in the case of the esters (R⁶ ≠ H in the formula (Ib)), the acids are reacted with the corresponding alcohols by customary methods, and **in that**, if appropriate, the dipeptides are converted into their salts.

4. Dipeptides according to Claim 1 or 2 for treatment of diseases, in particular of mycoses.

5. Medicaments comprising dipeptides according to Claim 1 or 2 for combating diseases, in particular mycoses.

6. Use of dipeptides according to Claim 1 or 2 for preparing medicaments, in particular for controlling mycoses.

## Revendications

1. Dipeptide constitué d'un α-aminoacide de formule générale (Ia) 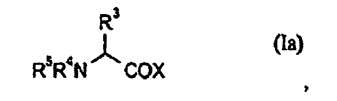 et d'un cyclopentane-β-aminoacide de formule générale (Ib) 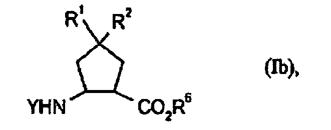 formules dans lesquelles
R³ désigne un reste méthyle ou un groupe de formule
- CH (CH₃) CH₂CH₃ ,
R⁴ et R⁵ représentent l'hydrogène, ou bien
R³ et R⁴ forment ensemble un reste de formule - (CH₂)₃-,
R⁵ représente l'hydrogène et
X représente la portion de la liaison covalente de l'α-aminoacide et du cyclopentane-β-aminoacide,
R¹ et R² représentent l'hydrogène, ou bien
R¹ et R² forment ensemble un reste de formule =CH₂,
R⁶ représente l'hydrogène et
Y représente la portion de la liaison covalente de
l'α-aminoacide et du cyclopentane-β-aminoacide, et ses sels.

2. Dipeptide suivant la revendication 1, choisi dans le groupe consistant en :
(a) acide 1,2-cis-2-(S)-isoleucyl-amino-4-méthylènecyclopentane-1-carboxylique et
(b) acide 1,2-cis-2-(S)-alanyl-amino-4-méthylènecyclopentane-1-carboxylique et leurs sels.

3. Procédé de production de dipeptides suivant l'une des revendications 1 et 2, **caractérisé en ce qu'**on transforme tout d'abord des composés de formule générale (II) 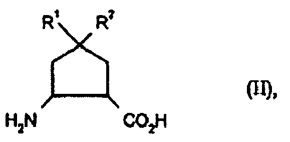 dans laquelle
R¹ et R² ont les définitions indiquées ci-dessus,
par réaction avec des aminoacides protégés de formule générale (III) 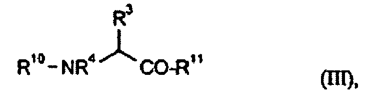 dans laquelle
R³ et R⁴ ont les définitions indiquées ci-dessus,
R¹⁰ représente un groupe protégeant la fonction amino, et
R¹¹ représente un groupe protecteur activateur usuel dans
la chimie des peptides, avantageusement le reste ester d'hydroxysuccinimide, ou bien
R¹⁰ et R¹¹ forment ensemble le groupement 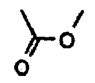 en composés de formule générale (IV) 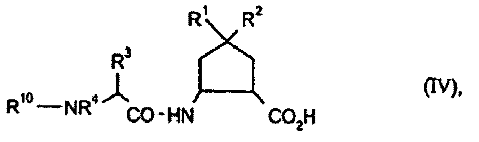 dans laquelle
R¹, R², R³, R⁴ et R¹⁰ ont les définitions indiquées ci-dessus, dans des solvants et en présence d'une base,
puis on élimine le groupe (R¹⁰) protégeant la fonction amino, on sépare éventuellement les stéréoisomères, dans le cas des esters (R⁶ ≠ H dans la formule (Ib)), on fait réagir les acides selon des modes opératoires usuels avec les alcools correspondants, et **en ce qu'**on transforme éventuellement les dipeptides en leurs sels.

4. Dipeptides suivant l'une des revendications 1 et 2, destinés au traitement de maladies, notamment de
mycoses.

5. Médicament, contenant des dipeptides suivant l'une des revendications 1 et 2 destinés à combattre des maladies, en particulier des mycoses.

6. Utilisation de dipeptides suivant l'une des revendications 1 et 2, pour la préparation de médicaments, en particulier destinés à combattre des mycoses.
